(19) Europäisches Patentamt
European Patent Office
Office européen des brevets

(11) Veröffentlichungsnummer: **0 014 264**
**B1**

# (12) EUROPÄISCHE PATENTSCHRIFT

(45) Veröffentlichungstag der Patentschrift: **20.01.82**

(51) Int. Cl.³: **C 07 C 119/12**

(21) Anmeldenummer: **79105413.3**

(22) Anmeldetag: **31.12.79**

## (54) Verfahren zur Herstellung von Schiff'schen Basen durch Umsetzung von aromatischen Aminen mit aliphatischen Ketonen in Gegenwart von Katalysatoren.

(30) Priorität: **18.01.79 DE 2901863**

(43) Veröffentlichungstag der Anmeldung: **20.08.80 Patentblatt 80/17**

(45) Bekanntmachung des Hinweises auf die Patenterteilung: **20.01.82 Patentblatt 82/3**

(84) Benannte Vertragsstaaten: **BE DE FR GB IT NL**

(56) Entgegenhaltungen:
**DE-A1-2 525 295**
**DE-C 301 121**
**GB-A-1 357 256**
**US-A-3 414 616**

(73) Patentinhaber: **BAYER AG, Zentralbereich Patente, Marken und Lizenzen, D-5090 Leverkusen 1, Bayerwerk (DE)**

(72) Erfinder: **George, Joachim, Dr., Tannenweg 1, D-5090 Leverkusen 31 (DE)**

**EP 0 014 264 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG.

# Verfahren zur Herstellung von Schiff'schen Basen durch Umsetzung von aromatischen Aminen mit aliphatischen Ketonen in Gegenwart von Katalysatoren

Die Erfindung betrifft ein Verfahren zur Herstellung von Schiff'schen Basen durch Umsetzung von aromatischen Aminen mit Ketonen in Gegenwart von Katalysatoren.

Die Kondensation von aromatischen Aminen mit Ketonen ist eine Gleichgewichtsreaktion. Sie wird zugunsten der Schiff'schen Base-Bildung verschoben, wenn man das entstehende Wasser der Reaktion entzieht. Dies geschieht in zweckmässiger Weise durch azeotrope Destillation mittels eines Schleppmittels.

Führt man die Reaktion ohne Zusätze durch, die die Wasserabspaltung fördern, entsteht in verhältnismässig langen Reaktionszeiten die gewünschte Schiff'sche Base nur in niedrigen Ausbeuten.

Die GB-PS 1 357 256 beschreibt die Reaktion von primären Aminen mit Ketonen in Gegenwart von Molekularsieben. Dieses Verfahren ist bereits in der DE-OS 2 525 295 als nachteilig beschrieben worden. So heisst es ebenda auf Seite 1, Absatz 2, dass bei Anwendung von Molekularsieben eine aufwendige Aufarbeitung notwendig ist, um das Endprodukt zu isolieren. Weiterhin ist die Wasseraufnahmefähigkeit der Molekularsiebe begrenzt und nach einer bestimmten Zeit ein Desorptionsschritt notwendig.

Die in der DE-OS zur Benutzung vorgeschlagenen sauren Ionenaustauscher sind gegenüber den erfindungsgemäss einsetzbaren Verbindungen teure Produkte, die darüber hinaus im Hinblick auf die Lehre der US-PS 2 533 723 nicht den nächstliegenden Stand der Technik darstellen.

Besagte US Patentschrift beschreibt Bleicherde als Katalysator für die Herstellung von Schiff'schen Basen durch Umsetzung von primären Aminen mit Katonen. Es hat sich gezeigt, dass der Einsatz dieser Katalysatoren nur zu einer Ausbeute von 78% führt.

Es wurde nunmehr gefunden, dass durch Zusatz bestimmter Verbindungen, die Wasserabspaltung und damit die Herstellung der Schiff'schen Base beschleunigt wird. Gleichzeitig erhält man das gewünschte Ausgangsprodukt in hoher Ausbeute.

Gegenstand der Erfindung ist somit ein Verfahren zur Herstellung der Schiff'schen Base der Formel (1)

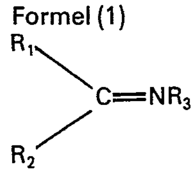
(1)

durch Umsetzung eines primären aromatischen Amins der Formel (2)

$H_2N-R_3$ (2)

mit einem Keton der Formel (3)

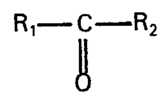

mit anschliessender Entfernung des gebildeten Wassers durch azeotrope Destillation, das dadurch gekennzeichnet ist, dass man die Umsetzung in Gegenwart von Calciumhydrogenphosphat, Apatit, Aluminiumoxid und/oder Aluminiumhydroxid durchführt.

Die Reaktion wird durch folgendes Formelschema verdeutlicht:

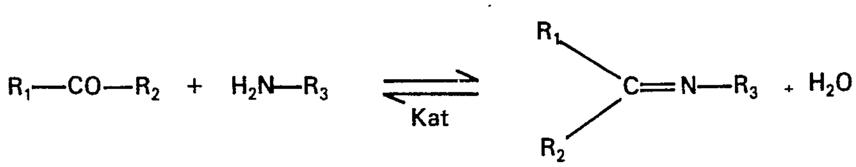


(3) (2) (1)

In obiger Formel (3) bedeutet: $R_1$ einen $C_1$–$C_3$-Alkylrest wie z.B. Methyl, Äthyl, Propyl, iso-Propyl, bevorzugt Methyl, Äthyl und iso-Propyl; $R_2$ einen geradkettigen oder verzweigten $C_2$–$C_9$-Alkylrest wie z.B. Äthyl, Propyl, iso-propyl, Butyl, iso-Butyl, tert.-Butyl, Pentyl, iso-Pentyl, Hexyl, iso-Hexyl, Heptyl, iso-Heptyl, Octyl, iso-Octyl, Nonyl, iso-Nonyl, bevorzugt Äthyl, Propyl, iso-Propyl, Butyl, iso-Butyl, tert.-Butyl; weiterhin können $R_1$ und $R_2$ miteinander verbunden sein und einen cyclischen $C_5$–$C_7$-Alkylring ergeben, wie z.B. Cyclohexyl oder Methylcyclohexyl.

Folgende Verbindungen seien im einzelnen beispielhaft aufgeführt:

Butanon-2, 3-Methylbutanon-2, 4-Methylpentanon-3, 5-Methylhexanon-2, 5-Methylhexanon-3, 4,4-Dimethylpentanon-2, Cyclohexanon, 4-Methylcyclohexanon.

In obiger Formel 2 bedeutet $R_3$ einen gegebenenfalls mit bis zu 3 $C_1$–$C_3$-Alkylgruppen substituierten Phenylrest wie Phenyl, Tolyl, Xylyl, Trimethylphenyl, Äthylphenyl, Diäthylphenyl oder einen Di- oder Triphenylaminrest, der gegebenenfalls ein- bis dreifach durch $C_1$–$C_3$-Alkyl oder Aminoreste substituiert sein kann, wobei die Substituenten sich alle in dem Phenylring befinden, der nicht mit der $NH_2$-Gruppe verbunden ist. Allgemein kann dies durch folgende Formeln wiedergegeben werden:

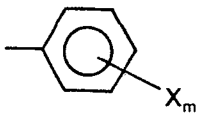

mit X = $C_1$–$C_3$-Alkyl und m = 0 bis 3, bevorzugt 0 bis 2 und

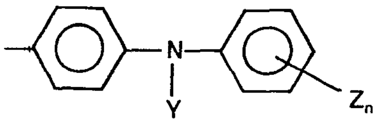

mit Z = $C_1$–$C_3$-Alkyl, Amino, Y = H, Phenyl, n = 0 bis 3, bevorzugt 0 bis 1.

Im einzelnen seien folgende Verbindungen beispielhaft aufgeführt:

Anilin
o-, m-, p-Toluidin
m-Phenylen-diamin
p-Phenylendiamin
4-Äthyl-anilin
4-Amino-diphenylamin
2-Methyl-4′-aminodiphenylamin
3-Methyl-4′-aminodiphenylamin
4-Methyl-4′-aminodiphenylamin
4,4′-Diaminotriphenylamin

Im Endprodukt der Formel 1 besitzen die Reste $R_1$, $R_2$ und $R_3$ die bereits angegebene Bedeutung. Folgende Schiff'sche Basen seien beispielhaft erwähnt:

N-1-methylpropyliden-phenylimin
N-1,3-dimethylbutyliden-phenylimin
N-1,4-dimethylpentyliden-4-tolylimin
N-Cyclohexyliden-phenylimin
N-Cyclohexyliden-4-tolylimin
N,N′-Bis(1,3-dimethylbutyliden)-1,4-diiminobenzol
N,N′-Bis(1,4-dimethylpentyliden)-1,4-diiminobenzol
N-1-methylpropyliden-4-iminodiphenylamin
N-1,3-dimethylbutyliden-4-iminodiphenylamin
N-1,4-dimethylpentyliden-4-iminodiphenylamin
N-Cyclohexyliden-4-iminodiphenylamin
N-1,3-dimethylbutyliden-4-imino-4′-methyldiphenylamin
N-1,3-dimethylbutyliden-4-imino-3′-methyldiphenylamin
N-1,3-dimethylbutyliden-4-imino-2′-methyldiphenylamin
N-1,4-dimethylpentyliden-4-imino-4-methyldiphenylamin
N,N′-Bis(1,3-dimethylbutyliden)-4,4′-diiminotriphenylamin

Als Verbindungen, die die Schiff'sche Basebildung erfindungsgemäss beschleunigen, werden aufgeführt:

1) Calciumhydrogenphosphat ($CaHPO_4$). Dieses Produkt sollte synthetisiert sein und wasserfrei aber nicht gebrannt sein.
2) Apatit [$Ca_5(PO_4)_3(COH)$]
3) Aluminiumhydroxide und -oxide. Diese Produkte sollten zweckmässigerweise nicht geglüht sondern nur getrocknet sein. Weiterhin sollten sie in verdünnter, warmer Salzsäure (5% bei 70 °C) löslich sein.

Bevorzugt können Verbindungen $CaHPO_4$, $Ca_5(PO_4)_3OH$, $AlOOH$, $Al_2O_3$, $Al_2(SiO_2)_3$ (K, Na, Ca) eingesetzt werden.

Die erfindungsgemäss einsetzbaren Verbindungen 1 bis 4 beschleunigen die Reaktion, erhöhen den Umsatz und drängen Nebenreaktionen wie die Selbstkondensation der Ketone zurück. Die Verordungen 1 bis 4 können auch im Gemisch verwendet werden. Sie werden bevorzugt in Mengen von 0,1 bis 5 Gew.-%, besonders bevorzugt von 0,5 bis 2 Gew.-%, bezogen auf das Amin eingesetzt.

Das Molverhältnis von Amin (2) zu Keton (3) beträgt bevorzugt 1:1 bis 1:10, besonders bevorzugt 1:1 bis 1:3.

Die Reaktionstemperatur beträgt im allgemeinen 50 bis 150 °C, bevorzugt 80 bis 130 °C.

Als Schleppmitel wie auch als Lösungsmittel wird bevorzugt das Keton selbst verwendet. Als Hilfsschleppmittel oder -lösungsmittel können Kohlenwasserstoffe, wie n- und iso-Hexane, -Heptane, -Oktane, Cyclohexan, Methylcyclohexane, Benzol, Toluol, Xylole oder Chlorkohlenwasserstoff wie Dichlormethan, Dichloräthan, Trichlormethan (Chloroform), Trichloräthylen, eingesetzt werden. Als weitere Hilfslösungsmittel können Alkanole wie 4-Methylpentanol-2, Cyclohexanol eingesetzt werden. Das Gewichtsverhältnis Keton zu Hilfslösungs- oder Schleppmittel beträgt bevorzugt 1:0,2 bis 1:5.

Die nach dem erfindungsgemässen Verfahren erhaltenen Schiff'schen Basen stellen wertvolle Zwischenprodukte für die Herstellung von substituiertem Phenylendiamin dar, die beispielsweise als Alterungsschutzmittel für Kautschuke eingesetzt werden können.

Beispiel 1
(2,4-Dimethyl-butylen-4-iminodiphenylamin)

1 Mol 4-Aminodiphenylamin wird mit 2,5 Mol Methylisobutylketon in Gegenwart von 5 g Calciumhydrogenphosphat am Rückfluss über einen Wasserabscheider zum Sieden erhitzt. Es stellt sich eine Sumpftemperatur von 130 °C ein. Nach 3 Stunden sind 17 g Wasser abgespalten. Der Umsatz beträgt 92%.

Die Durchführung der Reaktion ohne Katalysator unter den gleichen Bedingungen liefert nach 6 Stunden nur 62% Umsatz. Eine weitere Fortführung der Reaktion ergibt keine Umsatzsteigerung.

Beispiel 2
(2,5-Dimethyl-pentylen-4-iminodiphenylamin)

Unter den gleichen Bedingungen wie in Beispiel 1 angegeben wird statt Methylisobutylketon Methylisoamylketon eingesetzt. Es stellte sich eine Sumpftemperatur von 120 °C ein. Der Umsatz beträgt 85%.

**Patentansprüche**

1. Verfahren zur Herstellung von Schiff'schen Basen der allgemeinen Formel 1

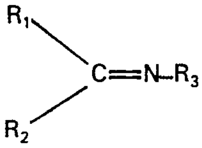

(1)

in der
$R_1$ einen $C_1$–$C_3$-Alkylrest,
$R_2$ einen $C_2$–$C_9$-Alkylrest oder
$R_1$ und $R_2$ zusammen einen cyclischen $C_5$–$C_7$-Alkylring bedeuten und
$R_3$ einen Rest der Formeln

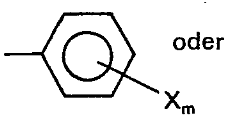

oder

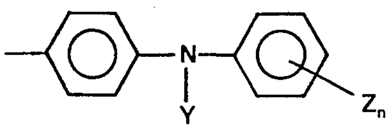

darstellt, wobei
X einen $C_1$–$C_3$-Alkylrest,
m 0 bis 3,
Y Wasserstoff oder Phenyl
Z einen $C_1$–$C_3$-Alkylrest und/oder Amino und
n 0 bis 3
bedeuten,
durch Umsetzung eines Amins der Formel 2

$H_2N$-$R_3$ (2)

mit Keton der Formel 3

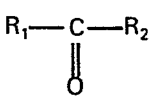

in welcher $R_1$, $R_2$ und $R_3$ die bereits angegebene Bedeutung besitzen und anschliessende Entfernung des gebildeten Wassers durch azeotrope Destillation, dadurch gekennzeichnet, dass man die Umsetzung in Gegenwart von Calciumhydrogenphosphat, Apatit, Aluminiumoxid und/oder Aluminiumhydroxid durchführt.

2. Verfahren nach Anspruch 1, dadurch gekennzeichnet, dass das Molverhältnis Amin zu Keton 1:1 bis 1:10 beträgt.

3. Verfahren nach Anspruch 1, dadurch gekennzeichnet, dass das Molverhältnis Amin zu Keton 1:1 bis 1:3 beträgt.

4. Verfahren nach den Ansprüchen 1 bis 3, dadurch gekennzeichnet, dass die Katalysatoren in Mengen von 0,1 bis 5 Gew.-%, bezogen auf das Amin, eingesetzt werden.

5. Verfahen nach den Ansprüchen 1 bis 4, dadurch gekennzeichnet, dass die Katalysatoren in Mengen von 0,5 bis 2 Gew.-%, bezogen auf das Amin, eingesetzt werden.

6. Verfahren nach Ansprüchen 1 bis 5, dadurch gekennzeichnet, dass die Reaktionstemperatur 50 bis 150 °C beträgt.

7. Verfahren nach den Ansprüchen 1 bis 5, dadurch gekennzeichnet, dass die Reaktionstemperatur 80 bis 130 °C beträgt.

**Claims**

1. A process for the preparation of Schiff's bases of the general formula (1)

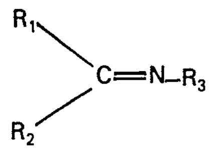

(1)

wherein
$R_1$ denotes a $C_1$–$C_3$ alkyl group,
$R_2$ denotes a $C_2$–$C_9$ alkyl group, or
$R_1$ and $R_2$ together denote a cyclic $C_5$–$C_7$ alkyl ring, and
$R_3$ denotes a group of the formulae

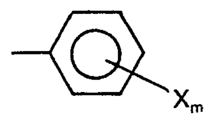

or

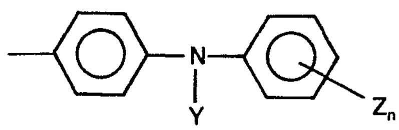

wherein
X denotes a $C_1$–$C_3$ alkyl group,
m represents 0 to 3,
Y denotes hydrogen or phenyl,
Z denotes a $C_1$–$C_3$ alkyl group and/or amino, and
n represents 0 to 3
by the reaction of an amine of the formula (2):

$H_2N$-$R_3$

with a ketone of the formula (3):

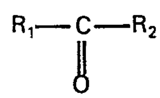

wherein $R_1$, $R_2$ and $R_3$ have the meanings already indicated, and the subsequent removal of the water formed by azeotropic distillation, characterised in that the reaction is carried out in the presence of calcium hydrogen phosphate, apatite, aluminium oxide and/or aluminium hydroxide.

2. A process according to Claim 1, characterised in that the molar ratio of the amine to the ketone is in the range of from 1:1 to 1:10.

3. A process according to Claim 1, characterised in that the molar ratio of the amine to the ketone is in the range of from 1:1 to 1:3.

4. A process according to Claims 1 to 3, characterised in that the catalysts are used in quantities of from 0.1 to 5% by weight, based on the amine.

5. A process according to Claims 1 to 4, characterised in that the catalysts are used in quantities of from 0.5 to 2% by weight, based on the amine.

6. A process according to Claims 1 to 5, characterised in that the reaction temperature is in the range of from 50 to 150 °C.

7. A process according to Claims 1 to 5, characterised in that the reaction temperature is in the range of from 80 to 130 °C.

**Revendications**

1. Procédé de fabrication de bases de Schiff de formule générale 1

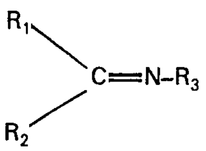

(1)

dans laquelle
$R_1$ représente un radical alcoyle en $C_1$–$C_3$,
$R_2$ un radical alcoyle en $C_2$–$C_9$ ou
$R_1$ et $R_2$ signifient ensemble un noyau alcoyle cyclique en $C_5$–$C_7$ et
$R_3$ un radical de formules

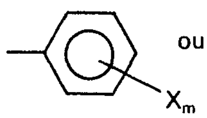

ou

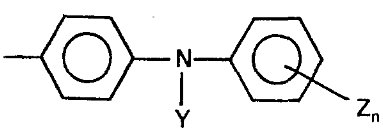

où
X représente un radical alcoyle en $C_1$–$C_3$
m est 0 à 3,
Y représente de l'hydrogène ou un phényle
Z représente un radical alcoyle en $C_1$–$C_3$ et/ou un amino et
n est 0 à 3,
par réaction d'une amine de formule 2

$H_2N$–$R_3$ (2)

avec une cétone de formule 3

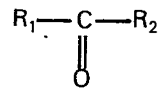

dans laquelle $R_1$, $R_2$ et $R_3$ possèdent la signification déjà indiquée, et par élimination consécutive de l'eau formée par distillation azéotropique, caractérisé en ce qu'on exécute la réaction en présence d'hydrogénophosphate de calcium, d'apatite, d'oxyde d'aluminium et/ou d'hydroxyde d'aluminium.

2. Procédé selon la revendication 1, caractérisé en ce que le rapport molaire de l'amine à la cétone est de 1:1 à 1:10.

3. Procédé selon la revendication 1, caractérisé en ce que le rapport molaire de l'amine à la cétone est de 1:1 à 1:3.

4. Procédé selon les revendications 1 à 3, caractérisé en ce que les catalyseurs sont utilisés en des quantités de 0,1 à 5% en poids par rapport à l'amine.

5. Procédé selon les revendications 1 à 4, caractérisé en ce que les catalyseurs sont utilisés en des quantités de 0,5 à 2% en poids par rapport à l'amine.

6. Procédé selon les revendications 1 à 5, caractérisé en ce que la température de réaction s'élève à 50–150 °C.

7. Procédé selon les revendications 1 à 5, caractérisé en ce que la température de réaction s'élève à 80–130 °C.